(19) Europäisches Patentamt
European Patent Office
Office européen des brevets

(11) **EP 4 617 642 A1**

(12) **EUROPEAN PATENT APPLICATION**

(43) Date of publication:
**17.09.2025 Bulletin 2025/38**

(21) Application number: **25162212.2**

(22) Date of filing: **07.03.2025**

(51) International Patent Classification (IPC):
**G01N 21/31** (2006.01)   **G01N 21/35** (2014.01)
**G01N 21/51** (2006.01)

(52) Cooperative Patent Classification (CPC):
**G01N 21/9027; G01N 21/31; G01N 21/51;**
G01N 21/3563; G01N 21/359; G01N 2021/4769;
G01N 2021/8845; G01N 2201/08

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.03.2024 JP 2024038567**

(71) Applicant: **Konica Minolta, Inc.**
**Tokyo 100-7015 (JP)**

(72) Inventor: **NAGAI, Koji**
**Tokyo 100-7015 (JP)**

(74) Representative: **Gille Hrabal**
**Partnerschaftsgesellschaft mbB**
**Patentanwälte**
**Brucknerstraße 20**
**40593 Düsseldorf (DE)**

(54) **QUALITY INSPECTION APPARATUS, QUALITY INSPECTION METHOD, AND PROGRAM**

(57) A quality inspection apparatus (1) is configured to inspect quality of an inspection target sample (S) filled in a transparent container (B). The inspection target sample is a lyophilized product or a powdery pharmaceutical product. The quality inspection apparatus includes: an illuminator (13) that illuminates a bottom surface of the transparent container; a multispectral light receiver (12) that obtains an image of a lateral surface of the transparent container; and a hardware processor (21). The hardware processor calculates an absorption spectrum at multiple wavelengths, based on a light reception result by the multispectral light receiver, and detects an inspection target component contained in the inspection target sample, based on the calculated absorption spectrum.

FIG.2

Processed by Luminess, 75001 PARIS (FR)

EP 4 617 642 A1

**Description**

Technical Field

The present invention relates to a quality inspection apparatus, a quality inspection method, and a program.

Description of Related Art

In the production of lyophilized products or powdery pharmaceutical products, moisture may remain in the products owing to insufficient drying. A hydrolysis reaction may occur between the moisture and active ingredients and affect the preservation stability of the products. Therefore, the products are to be produced so that the amount of water in the products is sufficiently small.

In the production of lyophilized products or powdered pharmaceutical products, the components of the products are to be examined (e.g., the amount of water). Japanese Patent No. 3351910 and International Publication No. 2007-063840 disclose techniques of inspecting lyophilized products or pharmaceutical products.

In the production of lyophilized products or powdered pharmaceutical products, some products may contain the amount of water that is not as designed, owing to the influence of the increased size of a production apparatus (lyophilization chamber). There may also be variations in the amount of water among the products. Therefore, in the production of the pharmaceutical products, non-destructive 100% inspection is desired.

Conventionally, components of lyophilized products/pharmaceutical products are inspected by destructive sampling inspection at the time of production. There is a concern about whether the destructive sampling inspection can thoroughly detect defective products. On the other hand, conventional non-destructive appearance inspection cannot directly measure the amount of components and internal defects of lyophilized products or pharmaceutical products.

Detailed inspection of the product quality is desired in the production of lyophilized products or powdery pharmaceutical products.

In order to address the above-described issue, an object of the present invention is to provide a quality inspection apparatus, a quality inspection method, and a program capable of performing detailed inspection of the quality of lyophilized products or powdered pharmaceutical products.

SUMMARY OF THE INVENTION

To achieve at least one of the abovementioned objects, according to an aspect of the present invention, a quality inspection apparatus reflecting one aspect of the present invention is configured to inspect quality of an inspection target sample filled in a transparent container, the inspection target sample being a lyophilized product or a powdery pharmaceutical product, the quality inspection apparatus including: an illuminator that illuminates a bottom surface of the transparent container; a multispectral light receiver that obtains an image of a lateral surface of the transparent container; and a hardware processor that calculates an absorption spectrum at multiple wavelengths, based on a light reception result by the multispectral light receiver, and that detects an inspection target component contained in the inspection target sample, based on the calculated absorption spectrum.

BRIEF DESCRIPTION OF THE DRAWINGS

The advantages and features provided by one or more embodiments of the invention will become more fully understood from the detailed description given hereinafter and the appended drawings which are given by way of illustration only, and thus are not intended as a definition of the limits of the present invention, and wherein:

FIG. 1 is a block diagram of a quality inspection apparatus according to the present embodiment;
FIG. 2 is a perspective view of an inspection unit;
FIG. 3 illustrates a configuration of the inspection unit;
FIG. 4 illustrates an example of light scattering properties;
FIG. 5 is a flowchart as an example of a quality inspection process;
FIG. 6 is a flowchart as an example of an absorption spectrum calculation process;
FIG. 7 illustrates an example of an evaluation position;
FIG. 8 illustrates an example of an absorption spectrum;
FIG. 9 illustrates an example of comparison between a reference absorption spectrum and an absorption spectrum of an inspection target sample;
FIG. 10 illustrates an example of comparison between absorption spectra;
FIG. 11A illustrates a case where the illumination region of an illumination unit is widened; and

FIG. 11B illustrates a case where the illumination region of the illumination unit is narrowed.

DETAILED DESCRIPTION

Hereinafter, one or more embodiments of the present invention will be described with reference to the drawings. However, the scope of the invention is not limited to the disclosed embodiments.

[Configuration example of quality inspection apparatus 1]

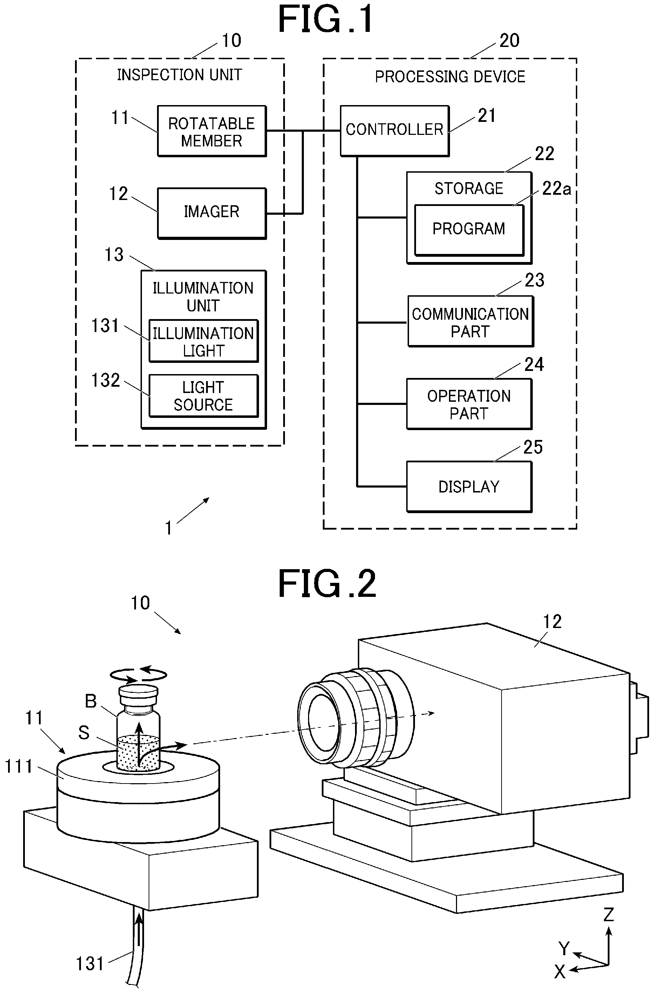

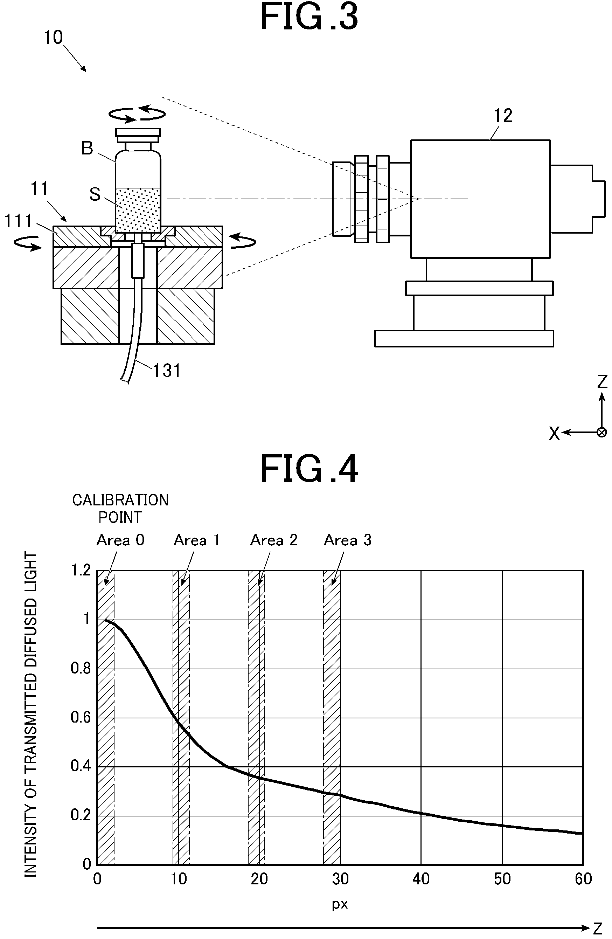

FIG. 1 is a block diagram of a quality inspection apparatus according to the present embodiment. The quality inspection apparatus 1 is configured to inspect the quality of an inspection target sample. The inspection target sample is a lyophilized product or a powdery pharmaceutical product.
As illustrated in FIG. 1, the quality inspection apparatus 1 includes an inspection unit 10 and a processing device 20. FIG. 2 is a perspective view of the inspection unit 10. FIG. 3 illustrates a configuration of the inspection unit 10. In FIG. 2 and FIG. 3, the X-axis direction and the Y-axis direction are two horizontal directions orthogonal to each other, and the vertical direction orthogonal to the X-axis and the Y-axis is defined as the Z-axis direction.
The inspection unit 10 includes a rotatable member 11, an imager 12 as a multispectral light receiver, and an illumination unit 13.
The rotatable member 11 rotates the sample stage 111 in a horizontal plane (XY plane) by the drive of a not-illustrated motor. On the sample stage 11, a transparent container B filled with a sample S is placed. The sample S is a lyophilized product or a powdered pharmaceutical product that is an inspection target of the quality inspection apparatus 1.
In the present embodiment, the quality inspection apparatus 1 is installed in a production line (inline) for producing lyophilized products or powdery pharmaceutical products. The inspection unit 10 includes a conveyance mechanism that conveys the transparent container B to the sample stage 111.
For another example, the quality inspection apparatus 1 may be installed off-line in a laboratory as a benchtop apparatus. In this case, the inspection unit 10 may not include a conveyance mechanism for conveying the transparent container B to the sample stage 111.
The imager 12 is, for example, a hyperspectral camera or a multichannel spectrometer. The imager 12 captures an image of the lateral surface of the transparent container B that is placed on the sample stage 111 and rotated by the rotatable member 11.
In the present embodiment, the sample S is imaged by the imager 12 while being rotated once. Therefore, the push-broom method is optimal as the spectroscopic principle of the hyperspectral camera as the imager 12. However, the spectroscopic principle of the hyperspectral camera may be a wavelength scanning type (e.g., a Fabry-Perot type) or a snapshot type. If it is possible to limit the imaging wavelength range of the imager 12 to a predetermined range, the imager 12 may be a multispectral camera.
One frame by the imaging of the imager 12 corresponds to one revolution (rotation) of the sample S.
The imager 12 performs imaging by separating wavelengths of light into multiple wavelength bands. Specifically, the imager 12 generates a data cube constituted of superposed two-dimensional (Y and y axes) plane images of the sample S (imaging target) of the respective spectral wavelength bands.
Thus, the imager 12 measures, in all directions of the sample S, the intensity of transmitted diffused light and light scattering properties generated by illuminating the sample S with the illumination light of the illumination unit 13. The imager 12 outputs the result of measuring the intensity of transmitted diffused light and light scattering properties to the processing device 20.
FIG. 4 illustrates details of light scattering properties obtained by imaging one revolution of the sample S. In the example illustrated in FIG. 4, the horizontal axis indicates pixels (px) in the Z-axis direction of one frame, and the vertical axis indicates the measured intensity of transmitted diffused light.
As shown in FIG. 4, the light scattering property exhibits a peak at a specific position (Area 0). On the Z-axis positive direction side of Area 0, the light scattering properties gradually attenuate in the Z-axis positive direction owing to the scattering properties, absorption properties, and so forth of the sample. The light scattering properties vary depending on the product type of the sample S and the production process.
In the examples illustrated in Figs. 2 and 3, the inspection unit 10 includes one imager 12. The inspection unit 10 may include two or more imagers 12. With multiple imagers 12, multiple samples S can be simultaneously imaged to increase the tact time and the inspection speed in the quality inspection process of the sample S.
It is desirable that the imaging wavelength range of the imager 12 include the near-infrared band because the near-infrared band is transmitted relatively highly and can acquire characteristic absorption peaks of components, such as active ingredients, excipients, and moisture contained in the sample S. In the present embodiment, the imaging wavelength range of the imager 12 is 900 to 1700 nm.
The illumination unit 13 includes a light member 131 and a light source 132. The illumination unit 13 irradiates, with illumination light, the bottom surface of the transparent container B filled with the sample S.

The light member 131 is constituted of optical fibers bundled together. The light member 131 guides illumination light emitted by the light source 132 to the tip end of the light member 131 (the end at the bottom surface side of the transparent container B). The light member 131 emits illumination light from the tip end surface toward the bottom surface of the transparent container B.

It is preferable that the light source 132 be a halogen lamp that has a broad wavelength range and a relatively high light intensity. The light source 132 may be a light emitting diode (LED).

The processing device 20 is, for example, a personal computer. The processing device 20 is connected to the rotatable member 11 and the imager 12 of the inspection unit 10 via not-illustrated wires and controls operation of the rotatable member 11 and the imager 12. The processing device 20 may be connected to the illumination unit 13 via wires to control the operation of the illumination unit 13.

The processing device 20 includes a controller 21 (hardware processor), a storage 22, a communication part 23, an operation part 24, and a display 25.

The controller 21 includes a processor, such as a central process unit (CPU) or a micro process unit (MPU), and a memory, such as a random access memory (RAM), for example. The controller 21 executes programs 22a stored in a memory, such as a RAM, or the storage 22 to execute various processes including the quality inspection process of the sample S.

The storage 22 includes, for example, a storage module, such as a hard disk drive (HDD), a solid state drive (SSD), a read only memory (ROM), and/or a RAM. The storage 22 stores, for example, a system program, an application program, and various kinds of data. Specifically, the storage 22 stores a program 22a for executing the quality inspection process of the sample S.

The communication section 23 includes, for example, a network interface card (NIC), a communication module including a receiver and a transmitter, and the like. The communication section 23 communicates various kinds of information and data with an external device or the like connected via a network such as the Internet.

The operation part 24 includes, for example, a mouse, a keyboard, switches, and buttons. The operation part 24 may be a touch screen combined with the display 25 or an interface that receives sound inputs, for example. The operation part 24 receives a command according to various input operations from a user, converts the received command into an operation signal, and outputs the operation signal to the controller 21.

The display 25 is, for example, a liquid crystal display or an organic electro luminescence (EL) display. The display 25 displays contents, based on display data output by the controller 21.

The quality inspection apparatus 1 configured as described above can be easily incorporated into an existing appearance inspection apparatus. For example, a benchtop diffuse reflectance spectroscopic analyzer may not be incorporated in line owing to a limited space. On the other hand, the quality inspection apparatus 1 can be easily incorporated into an existing appearance inspection apparatus by using the imager 12 and the illumination unit 13 instead of a machine vision camera of the existing appearance inspection apparatus.

[Operation of quality inspection apparatus 1]

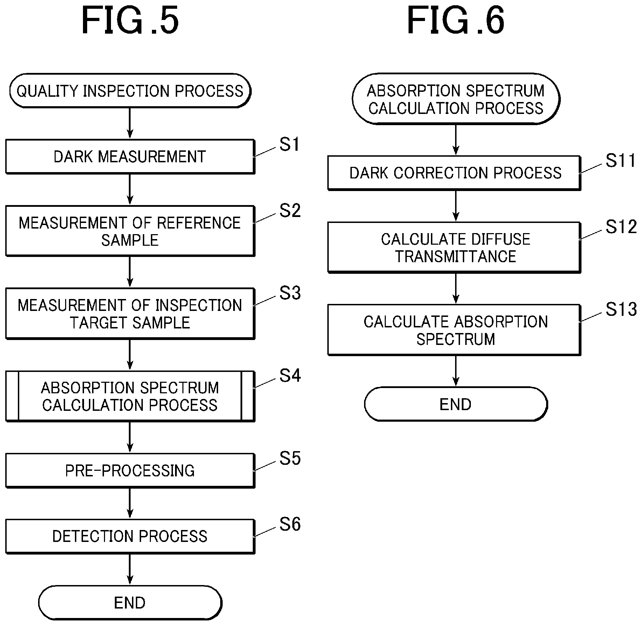

Next, the operation of the quality inspection apparatus 1 according to the present embodiment will be described. FIG. 5 is a flowchart of an example of the quality inspection process in which the quality inspection apparatus 1 inspects the quality of the sample S.

(Quality inspection process)

The user performs imaging with the imager 12 of the quality inspection apparatus 1 while blocking light. Thus, the imager 12 performs dark measurement and outputs dark data as the measurement result to the processing device 20 (step S1).

Next, with the imager 12, the user captures an image of a sample as a reference of quality inspection (hereinafter called a reference sample). Thus, the imager 12 performs measurement of the reference sample and outputs the measurement data of the reference sample to the processing device 20 (step S2).

The imager 12 generates an image, based on the obtained signal intensity of the illumination light emitted by the illumination unit 13, absorbed and scattered inside the sample, and having passed through the optical path to the imager 12. In the measurement of the reference sample, the signal of an object having a known absorption property and a known scattering property may be obtained in advance, and the signal may be used as a reference value. The reference sample may be a standard white plate made of polytetrafluoroethylene (PTFE) or may be a predetermined sample similar to the measurement sample, for example.

The measurement of the reference sample in step S2 includes calibration at the time of activation of the imager 12.

Next, with the imager 12, the user captures an image of one revolution of the sample S (inspection target sample) placed on the sample stage 111. Thus, the imager 12 measures the intensity of transmitted diffused light and the light scattering properties for one revolution of the inspection target sample, and outputs the measurement data to the processing device 20 (step S3).

Instead of performing measurement for one revolution of the inspection target sample, the imager 12 may perform measurement for only one frame of the inspection target sample or any range of the inspection target sample in step S3. In this case, the intensity of transmitted diffused light can be measured based on the measurement result of one frame, and the absorption spectrum can be calculated from the measurement data. If the inspection target sample has a crack, the intensity of transmitted diffused light and the light scattering properties may not be measured at the location of the crack. It is therefore preferable that the location of the crack be excluded from the target range of the detection process in step S6, which is described later.

Next, the controller 21 of the processing device 20 acquires the measurement data output by the imager 12. The controller 21 performs the absorption spectrum calculation process illustrated in FIG. 6, based on the acquired measurement data of the inspection target sample (step S4).

(Absorption spectrum calculation process)

The controller 21 performs a dark correction process (step S11). Specifically, the controller 21 subtracts the dark data obtained in step S1 from the measurement data of the inspection target sample. Thus, noise due to dark current in the imager 12 can be removed.

Next, the controller 21 calculates the diffuse transmittance of the inspection target sample, based on the measurement data after the dark correction process in step S11 (step S12). Following is the description of two methods for calculating the diffuse transmittance of the inspection target sample.

(Method 1 of calculating diffuse transmittance)

In the method 1 of calculating the diffuse transmittance, in step S3, the user images the inspection target sample at two or more measurement points in the Z-axis direction (the vertical direction of the transparent container B) using the imager 12.

The controller 21 performs the following calibration process on the light scattering properties, which are measurement data measured at two or more measurement points. Specifically, the controller 21 determines "px" corresponding to a peak, such as Area 0 in Fig. 4, to be the calibration point. The controller 21 divides the intensity of transmitted diffused light at the other measurement point(s) by the intensity of transmitted diffused light at the calibration point. The other measurement points are, for example, Area 1, Area 2, and Area 3 in FIG. 4. For another example, the controller 21 may divide the intensity of transmitted diffused light at the other measurement point by the average intensity of transmitted diffused light in a predetermined range including the calibration point. The controller 21 executes the calibration process for each frame captured by the imager 12. The measurement data after the calibration process indicates the diffuse transmittance of the inspection target sample. By the calibration process, the controller 21 normalizes the entire diffuse transmittances, based on the average diffuse transmittance in a predetermined range including the calibration point as 1. That is, among multiple pieces of measurement data at multiple measurement points (light reception results by the imager 12), the controller 21 calculates the relative intensity of transmitted diffused light (light reception amount) at one measurement point with reference to the intensity of transmitted diffused light at another measurement point as a reference; and, based on the relative light reception amount, the controller 21 calculates the absorption spectrum of the inspection target sample in step S13, which is described later.

By the calibration process, the influence of the light source 132 can be removed from the diffuse transmittance of the inspection target sample. Thus, in the detection process in step S6 described later, evaluation can be performed focusing only on the absorption of illumination light by the inspection target sample between the calibration point (reference position) and a different measurement point (evaluation position).

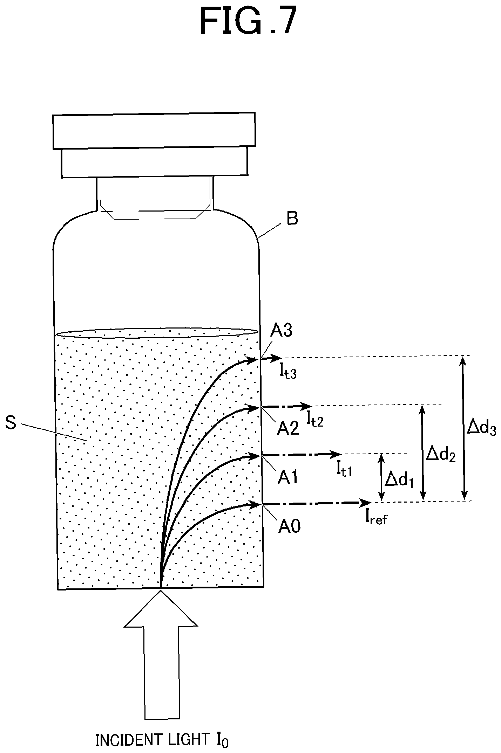

FIG. 7 illustrates the reference position A0 and the evaluation positions A1 to A3. As shown in FIG. 7, the distance between the reference position A0 and the evaluation position A1 is $\Delta d1$; the distance between the reference position A0 and the evaluation position A2 is $\Delta d2$; and the distance between the reference position A0 and the evaluation position A3 is $\Delta d3$. In this case, the diffuse transmittance Tn at an evaluation position An is obtained by the following expression (1) (Lambert-Beer law).

$$T_n = \frac{S - D}{W - D} = \frac{I_{tn}}{I_{ref}} = \frac{I_0 exp\{-\varepsilon c(\rho + \Delta d_n)\}}{I_0 exp(-\varepsilon c\rho)} = exp(-\varepsilon c\Delta d_n) \cdot \cdot \cdot (1)$$

S represents measurement data at an evaluation position An. W represents measurement data at the reference position A0. D represents dark data. E represents a molar extinction coefficient. C represents the concentration of the inspection target sample. P represents an average scattering distance (distance from the tip surface of the light member 131 to the reference position A0). Itn represents the light intensity measured at the evaluation position An. Iref represents the light intensity measured at the reference position A0. I0 represents the intensity of illumination light emitted by the light source 132. Δdn represents the distance from the reference position A0 to the evaluation position An.

As expressed by the expression (1), the influence of the light source 132 (I0) can be removed from the diffuse transmittance Tn of the inspection target sample. Thus, in the detection process of step S6 described later, evaluation can be made focusing on the influence of the components and the concentration of the inspection target sample. In the inspection process of step S6, evaluation can be made focusing on a predetermined evaluation position An of the inspection target sample.

In the calculation of the diffuse transmittance of the inspection target sample by the method 1 described above, the measurement of the reference sample in step S2 may be omitted.

(Method 2 of calculating diffuse transmittance)

In the method 2 of calculating diffuse transmittance, the controller 21 calculates the diffuse transmittance Tn at a predetermined evaluation position An by using the measurement data of the reference sample obtained in step S2 as "W" in the above expression (1). In the method 2, the detection process of step S6 described later can be performed based on the reference sample, and an abnormality in the inspection target sample can be detected.

In the expression (1), "W" may be measurement data of a predetermined sample or measurement data at a different evaluation position of the inspection target sample.

For another example, the quality inspection apparatus 1 may include a reference light receiver (not illustrated) that measures the amount of light emitted by the light source 132 of the illumination unit 13. In this case, in the method 2 of calculating the diffuse transmittance, the controller 21 uses the light reception amount of the light source 132 measured by the reference light receiver as "W" in the above expression (1) to calculate the diffuse transmittance Tn at a predetermined evaluation position An. That is, in step S13 described later, the controller 21 calculates the absorption spectrum of the inspection target sample, based on the light reception amount of the light source 132 measured by the reference light receiver.

Next, the controller 21 calculates absorbance, based on the diffuse transmittance of the inspection target sample calculated in step S12. The controller 21 calculates the absorbance at each wavelength of the transmitted diffused light to calculate the absorption spectrum (step S13) and ends the absorption spectrum calculation process. That is, the controller 21 calculates the absorption spectrum at multiple wavelengths, based on the measurement data by the imager 12 (the light reception result by the multispectral light receiver). The controller 21 serves as a calculator. Step S13 is a calculation step.

Following is the description of two methods for calculating the absorbance of the inspection target sample.

(Method 1 of calculating absorbance)

In the method 1 of calculating absorbance, the controller 21 calculates the absorbance Absn at a predetermined evaluation position An of the inspection target sample by the following expression (2).

$$Abs_n = -\log T_n = \varepsilon c \Delta d_n \quad \cdot \quad \cdot \quad \cdot \quad (2)$$

As in the above expression (2), the influence of the average scattering distance can be removed from the absorbance Absn of the inspection target sample.

Thus, in the inspection process of step S6 described later, evaluation can be made focusing on a predetermined evaluation position An of the inspection target sample.

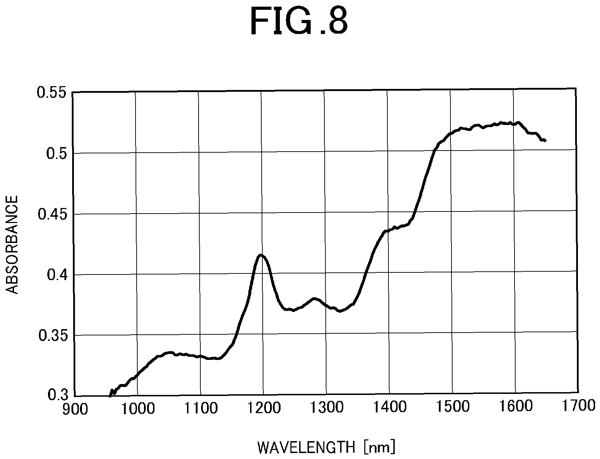

FIG. 8 illustrates an example of the absorption spectrum calculated based on the diffuse transmittance. In the example illustrated in FIG. 8, the horizontal axis indicates the wavelength of the measured transmitted diffused light, and the vertical axis indicates the calculated absorbance.

(Method 2 of calculating absorbance)

In the method 2 of calculating absorbance, the controller 21 calculates the absorbance, based on the light scattering property of the transmitted diffused light at each wavelength.

In the above expression (1), $\varepsilon c \Delta d_n$ is unknown in the measurement data obtained in Step S3. However, the light attenuation property of the diffuse transmittance Tn can be obtained from the measurement data obtained in step S3. The controller 21 estimates εc by fitting the light attenuation property of the diffuse transmittance Tn with an exponential function. Thus, the unit absorption spectrum Abs can be calculated.

For another example, the controller 21 may estimate εc in the above expression (2) by linearly approximating the attenuation property of the absorbance Absn.

For another example, the controller 21 may estimate the scattering coefficient and the absorption coefficient by using an appropriate model, such as a light diffusion theory based on a measurement approach of spatially resolved spectroscopy (SRS).

In using the light diffusion theory, the controller 21 obtains multiple pieces of spectral information (intensity of transmitted diffused light) that are spatially different by the SRS method. The controller 21 then substitutes the light attenuation property of the spectral information into a light diffusion equation and solves the simultaneous equations. Thus, the controller 21 calculates the scattering coefficient $\mu_s'$ and the absorption coefficient $\mu_a$. Thus, the scattering coefficient $\mu_s'$ and the absorption coefficient $\mu_a$ can be calculated, and the content of components in the inspection target sample can be precisely detected in the detection process in step S6 described later.

The following expression (3) is the light diffusion equation R (ρ) at the same diameter distance (average scattering distance) ρ in a state where the scattering directions are randomized at a certain depth Z0 in the scattering medium and virtual light sources are thereby generated according to the principle of multiple light scattering. The effective attenuation coefficient $\mu_{eff}$ is expressed by the following expression (4).

$$R(\rho) = z_0\left(\frac{1}{\rho} + \mu_{eff}\right)\frac{\exp(-\mu_{eff}\rho)}{2\pi\rho^2} \quad \cdot \cdot \cdot (3)$$

$$\mu_{eff} = \sqrt{3\mu_a(\mu_a + \mu_s')} \quad \cdot \cdot \cdot (4)$$

By calculating the scattering coefficient $\mu_s'$ and the absorption coefficient $\mu_a$ by the above expressions (3) and (4), the controller 21 performs fitting to the diffusion theory model as expressed by the following expressions (5) to (9). In the following, D represents a diffusion coefficient, and A represents an internal reflection parameter.

$$R(\rho) = \frac{a'}{4\pi}\left[\frac{1}{\mu_t'}\left(\mu_{eff} + \frac{1}{r_1}\right)\frac{\exp(-\mu_{eff}r_1)}{r_1^2} + \left(\frac{1}{\mu_t'} + 2Z_b\right)(\mu_{eff} + \frac{1}{r_2})\frac{\exp(-\mu_{eff}r_2)}{r_2^2}\right] \quad \cdot \cdot \cdot (5)$$

$$a' = \frac{\mu_s}{\mu_t'} \quad \cdot \cdot \cdot (6)$$

$$Z_b = 2AD \quad \cdot \cdot \cdot (7)$$

$$r_1 = \sqrt{z_0^2 + \rho^2} \quad \cdot \cdot \cdot (8)$$

$$r_2 = \sqrt{(z_0 + 2z_b)^2 + \rho^2} \quad \cdot \cdot \cdot (9)$$

Next, the controller 21 performs pre-processing on the absorption spectrum calculated in step S4 (step S5). The pre-processing includes, for example, noise processing, normalization, correction processing (e.g., baseline correction processing), standard normal variate (SNV) processing, smoothing by the Savitzky-Golay method, and differential processing.

Next, the controller 21 performs the detection process of detecting the inspection target component contained in the inspection target sample, based on the absorption spectrum on which the pre-processing in step S5 has been performed (step S6). The controller 21 serves as a detector. Step S6 is a detection step. Examples of the inspection target component include an active pharmaceutical ingredient, an excipient, and moisture.

When the components of the inspection target sample vary depending on parts of the sample, the controller 21 may perform the following process in step S6. Specifically, the controller 21 generates spectroscopic imaging data from the measurement data of the inspection target sample obtained in step S3. The controller 21 executes the detection process by using the generated spectroscopic imaging data.

Following is the description of three examples for detecting the inspection target component contained in the inspection target sample.

(Example 1 of detection process)

In the example 1 of the detection process, the controller 21 detects the inspection target component by determining whether the inspection target component is contained in the inspection target sample. For example, the controller 21 determines whether a desired active pharmaceutical ingredient (API) and excipient are contained in the inspection target sample. For another example, the controller 21 determines whether an unintended component is contained in the inspection target sample.

Specifically, the controller 21 compares the reference absorption spectrum with the absorption spectrum of the inspection target sample after the pre-processing of step S5. The controller 21 makes the determination in the example 1 of the detection process, based on the degree to which the absorption spectrum of the inspection target sample after the pre-processing in step S5 matches with the reference absorption spectra. The reference absorption spectra are related to components of multiple reference samples corresponding to product types.

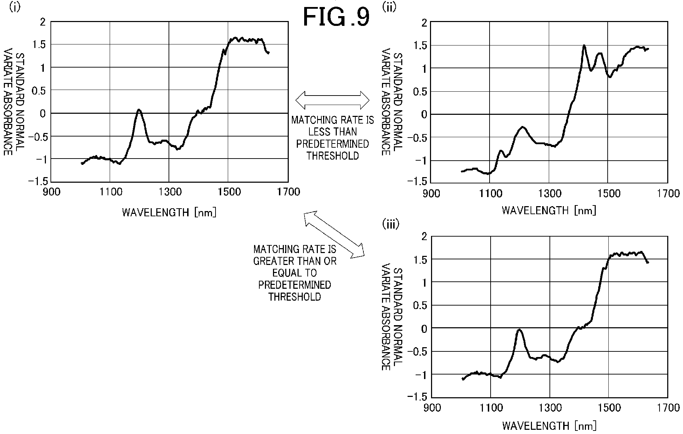

FIG. 9 illustrates an example of comparison between the reference absorption spectra and the absorption spectrum of the inspection target sample after the pre-processing in Step S5. The graph (i) in FIG. 9 shows the absorption spectrum of the inspection target sample after the pre-processing of step S5. The graphs (ii) and (iii) in FIG. 9 show the reference absorption spectra stored in the storage 22, for example. In the example of FIG. 9, the degree to which the graph (i) matches with the graph (ii) is less than a predetermined threshold, whereas the degree to which the graph (i) matches with the graph (iii) is greater than or equal to the predetermined threshold. In this case, the controller 21 determines that the inspection target sample does not contain the inspection target component contained in the reference sample corresponding to the graph (ii). The controller 21 determines that the inspection target sample contains the inspection target component contained in the reference sample corresponding to the graph (iii).

Thus, the controller 21 can detect contamination of the inspection target sample in the production process and unintended variations of components in the inspection target sample. This contributes to producing safe samples. By the above-described determination in the example 1 of the detection process, it is possible to identify a product (inspection target sample) containing unknown components as a specific product.

(Example 2 of detection process)

In the example 2 of the detection process, the controller 21 forms a calibration curve for the amount of a component contained in the inspection target sample (e.g., the amount of active ingredients, moisture content). Based on the calibration curve, the controller 21 quantifies the component contained in the inspection target sample.

Following is the description of a flow of building a machine learning model as a calibration curve of the moisture content in the inspection target sample.

First, the user prepares multiple samples having different water contents as correct value samples. Next, the user captures images of the prepared samples by the imager 12.

Next, the controller 21 performs a moisture quantitative analysis by chemical analysis (Karl Fischer method) on the measurement data of the samples obtained by the imager 12. Next, the controller 21 builds a calibration model by regression analysis, such as partial least squares regression (PLS), using the data set obtained by the moisture amount analysis (or data in a predetermined region of the data set). The controller 21 may build the calibration model by using principal component regression or deep learning, for example. When building the calibration model, the controller 21 may remove noise by cleansing the data set obtained by the water content analysis.

Next, the user captures an image of the inspection target sample by the imager 12. Next, the controller 21 performs steps S4 and S5 of the quality inspection process to calculate and correct the absorption spectrum, based on the measurement data of the inspection target sample obtained by the imager 12. Next, the controller 21 applies the absorption spectrum of the inspection target sample to the built calibration model and calculates the amount of the inspection target component contained in the inspection target sample (quantitative analysis). The controller 21 may perform quantitative analysis for each frame obtained by the imager 12 in addition to outputting one quantitative analysis result for each inspection target sample. Thus, the controller 21 may generate the distribution of the amount of the component contained in the inspection target sample.

(Example 3 of detection process)

In the example 3 of the detection process, the controller 21 detects an abnormal sample. Following is the description of the method of detecting an abnormal sample.

First, the user prepares multiple inspection target samples. Next, the user captures images of the prepared inspection target samples by the imager 12.

Next, the controller 21 performs steps S4 and S5 of the quality inspection process to calculate and correct the absorption spectra of the respective inspection target samples, based on the measurement data of the inspection target samples obtained by the imager 12. Next, the controller 21 compares the absorption spectra and detects an outlier absorption spectrum. The degree to which the shape of an outlier absorption spectrum matches with the shapes of the other absorption spectra is less than a predetermined threshold. Next, the controller 21 determines the inspection target sample corresponding to the absorption spectrum detected as an outlier to be an abnormal sample. The controller 21 may detect an outlier by statistical processing or deep learning.

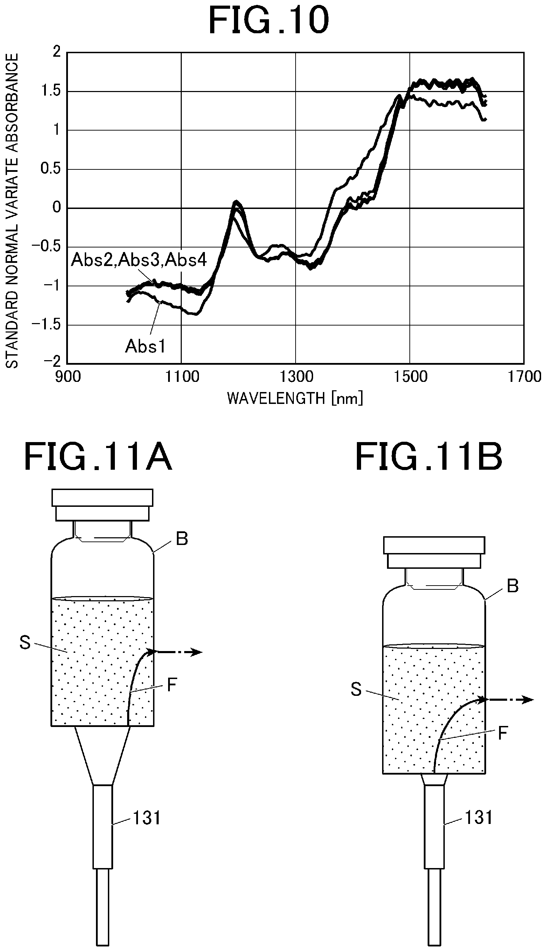

FIG. 10 illustrates an example of comparison among multiple absorbance spectra (Abs1 to Abs4). In the example of FIG. 10, the absorbance spectra Abs2 to Abs4 have substantially the same shape and overlap each other. The degree to which the shape of the absorption spectrum Abs1 matches with the shapes of the absorbance spectra Abs2 to Abs4 is less than a predetermined threshold. In this case, the controller 21 determines that the sample corresponding to the absorption spectrum Abs1 is an abnormal sample.

For another example, in the example 3 of the detection process, the controller 21 may detect an abnormal part in one sample. The method of detecting an abnormal part in one sample is described below.

First, the user captures images at multiple measurement positions of one sample with the imager 12.

Next, the controller 21 performs steps S4 and S5 of the quality inspection process to calculate and correct the absorption spectra of the respective measurement positions, based on the measurement data of the measurement positions obtained by the imager 12. Next, the controller 21 compares the absorption spectra and detects an outlier absorption spectrum. Next, the controller 21 determines the measurement position corresponding to the absorption spectrum detected as an outlier to be an abnormal part.

In the above quality inspection process, the transmitted diffused light is measured by the imager 12. By measuring the transmitted diffused light, it is possible to obtain information of the inside of the sample, instead of measuring only the components on the surface or near the surface of the sample by measuring reflected light. Thus, the quality of the sample can be inspected in detail. The amount of water in a lyophilized product or a powdered pharmaceutical product is very small. In the above quality inspection process, a minute amount of water can be precisely detected by measuring transmitted and diffused light that has a longer optical path.

In the above quality inspection process, the imager 12 measures the light scattering property, so that information related to an internal defect of the sample can be obtained. Therefore, an internal defect in the sample can be detected by the quality inspection process. The light scattering property depends on the scattering property of the sample and the absorption properties of components contained in the sample. Therefore, by the quality inspection process, the components of the sample can be detected.

[Others]

In the measurement in steps S2 and S3 of the quality inspection process, the illumination region of the bottom surface of the transparent container B illuminated by the illumination unit 13 may be adjusted. In the present embodiment, the illuminated region can be adjusted to the entire bottom surface of the transparent container B or only the vicinity of the center of the transparent container B, for example. Specifically, as shown in FIG. 11A, the illuminated region can be increased by increasing the distance between the tip end surface of the light member 131 and the bottom surface of the transparent container B. As shown in FIG. 11B, the illuminated region can be narrowed by shortening the distance between the tip end surface of the light member 131 and the bottom surface of the transparent container B. For another example, the illumination light may be guided by all the optical fibers constituting the light member 131 to hit the bottom surface of the transparent container B. Thus, the illuminated region can be increased. The illumination region can be narrowed by guiding the illumination light only with the optical fiber(s) at the center among the optical fibers constituting the light member 131 and irradiating the bottom surface of the transparent container B with the illumination light.

By adjusting the illumination region of the bottom surface of the transparent container B illuminated by the illumination unit 13, the length of the optical path F of the illumination light passing through the sample S can be adjusted. Thus, the length of the optical path F can be suitably determined for the absorbance of the sample S. For example, when the absorption of the illumination light by the sample S is significantly small, the amount of the illumination light absorbed by the sample S can be increased by increasing the length of the optical path F of the illumination light.

[Effects]

The quality inspection apparatus 1 of the present embodiment inspects the quality of an inspection target sample that is a lyophilized product or a powdery pharmaceutical product filled in a transparent container B. The quality inspection apparatus 1 includes the illumination unit 13 that illuminates the bottom surface of the transparent container B. The quality inspection apparatus 1 includes a multispectral light receiver (imager 12) that obtains an image of the lateral surface of the transparent container B. The quality inspection apparatus 1 includes a calculator (controller 21) that calculates absorption spectrum at multiple wavelengths, based on the light reception result by the multispectral light receiver. The quality inspection apparatus 1 includes a detector (controller 21) that detects the inspection target component contained in the inspection target sample, based on the absorption spectrum calculated by the calculator.

The quality inspection apparatus 1 configured as described above can detect the inspection target component contained in the inspection target sample without destroying the inspection target sample. Therefore, the quality inspection apparatus 1 can perform 100% inspection of lyophilized products or pharmaceutical products when these products are produced. That is, the quality of lyophilized products or powdery pharmaceutical products can be inspected in more detail.

In the present embodiment, the detector (controller 21) of the quality inspection apparatus 1 compares the absorption spectrum of the inspection target sample with the absorption spectrum of the reference sample to detect the inspection target component.

Thus, the controller 21 can detect contamination of the inspection target sample in the production process and unintended variations of components in the inspection target sample. This contributes to producing safe samples. It is possible to identify a product containing an unknown component (inspection target sample) as a specific product.

In the present embodiment, the detector (controller 21) of the quality inspection apparatus 1 calculates the amount of the inspection target component by applying the absorption spectrum of the inspection target sample to the calibration model built in advance.

Since the inspection target components can be quantitatively detected, the quality of the inspection target sample can be inspected in more detail.

According to the quality inspection apparatus 1 of the present embodiment, the inspection target component is at least one of an active pharmaceutical ingredient, an excipient, and moisture.

Since at least one of an active pharmaceutical ingredient, an excipient, and moisture contained in the inspection target sample can be detected, the quality of the inspection target sample can be inspected in more detail.

In the present embodiment, the detector (controller 21) of the quality inspection apparatus 1 compares the shapes of absorption spectra of multiple inspection target samples. When the degree to which a shape of an absorption spectrum of a specific inspection target sample matches with shapes of the other absorption spectra of the other inspection targets is less than a predetermined threshold, the detector (controller 21) determines that the specific inspection target sample is abnormal.

Thus, the quality of the inspection target sample can be easily performed without building a calibration curve or preparing absorption spectra of multiple reference samples in advance.

In the present embodiment, the multispectral light receiver (the imager 12) of the quality inspection apparatus 1 is a camera or a multi-channel spectrometer. The multispectral light receiver obtains images at multiple points in the vertical direction of the transparent container B. Based on a relative light reception amount with respect to another light reception amount as a reference among the light reception results at the multiple points, the calculator (controller 21) calculates the absorption spectrum.

Thus, the influence of the light source 132 can be removed from the diffuse transmittance of the inspection target sample. Thus, in the detection process of detecting the inspection target sample, evaluation can be performed focusing only on the absorption of illumination light by the inspection target sample between the calibration point (reference position) and a different measurement point (evaluation position).

According to the present embodiment, the quality inspection apparatus 1 includes a reference light receiver that measures the amount of light emitted by the light source 132 of the illumination unit 13. Based on the amount of emitted light measured by the reference light receiver, the calculator (controller 21) calculates the absorption spectrum. Thus, the inspection target sample can be inspected without performing measurement at multiple measurement points of the inspection target sample.

According to the quality inspection apparatus 1 of the present embodiment, the illumination region of the bottom surface of the transparent container B illuminated by the illumination unit 13 is adjustable.

Thus, the length of the optical path F can be suitably determined for the absorbance of the sample S.

According to the present embodiment, the multispectral light receiver (imager 12) of the quality inspection apparatus 1 is a hyperspectral camera.

The hyperspectral camera can obtain high-resolution images of an object at many wavelengths. Based on the measurement data obtained by the imaging, the quality of a lyophilized product or a powdery pharmaceutical product can be inspected in more detail.

Although the preferred embodiments of the present disclosure have been described in detail with reference to the accompanying drawings, the technical scope of the present disclosure is not limited to such examples. Furthermore, those to which various modification examples and improvements have been applied naturally belong to the technical scope of the present disclosure within the category of the technical idea described in the scope of the claims of those skilled in the art.

Although embodiments of the present invention have been described and shown in detail, the disclosed embodiments are made for purposes of illustration and example only and not limitation. The scope of the present invention should be interpreted by terms of the appended claims.

**Claims**

1. A quality inspection apparatus (1) configured to inspect quality of an inspection target sample (S) filled in a transparent container (B), the inspection target sample being a lyophilized product or a powdery pharmaceutical product, the quality inspection apparatus comprising:

   an illuminator (13) that illuminates a bottom surface of the transparent container;
   a multispectral light receiver (12) that obtains an image of a lateral surface of the transparent container; and
   a hardware processor (21) that calculates an absorption spectrum at multiple wavelengths, based on a light reception result by the multispectral light receiver, and that detects an inspection target component contained in the inspection target sample, based on the calculated absorption spectrum.

2. The quality inspection apparatus according to claim 1, wherein the hardware processor detects the inspection target component by comparing the absorption spectrum of the inspection target sample with an absorption spectrum of a reference sample.

3. The quality inspection apparatus according to claim 1, wherein the hardware processor calculates an amount of the inspection target component by applying the absorption spectrum to a calibration model built in advance.

4. The quality inspection apparatus according to claim 1, wherein the inspection target component is at least one of an active pharmaceutical ingredient, an excipient, and moisture.

5. The quality inspection apparatus according to claim 1, wherein:

   the hardware processor compares shapes of absorption spectra of multiple inspection target samples each of which is the inspection target sample, and
   when a degree to which a shape of an absorption spectrum of a specific inspection target sample matches with shapes of other absorption spectra of other inspection target samples is less than a predetermined threshold, the hardware processor determines that the specific inspection target sample is abnormal.

6. The quality inspection apparatus according to any one of claims 2 to 5, wherein:

   the multispectral light receiver is a camera or a multichannel spectrometer and obtains images at multiple points in a vertical direction of the transparent container, and
   based on a relative light reception amount with respect to another light reception amount as a reference among light reception results at the multiple points, the hardware processor calculates the absorption spectrum.

7. The quality inspection apparatus according to any one of claims 2 to 5, further comprising a reference light receiver that measures an amount of light emitted by a light source of the illuminator, wherein
   based on the amount of emitted light measured by the reference light receiver, the hardware processor calculates the absorption spectrum.

8. The quality inspection apparatus according to claim 1, wherein an illumination region on the bottom surface of the transparent container illuminated by the illuminator is adjustable.

9. The quality inspection apparatus according to claim 1, wherein the multispectral light receiver is a hyperspectral camera.

10. A quality inspection method to be executed by a quality control apparatus (1) configured to inspect quality of an inspection target sample (S) filled in a transparent container (B), the inspection target sample being a lyophilized product or a powdery pharmaceutical product, the quality inspection apparatus including: an illuminator (13) that illuminates a bottom surface of the transparent container; and a multispectral light receiver (12) that obtains an image of a lateral surface of the transparent container, the method comprising:

calculating an absorption spectrum at multiple wavelengths, based on a light reception result by the multispectral light receiver; and

detecting an inspection target component contained in the inspection target sample, based on the calculated absorption spectrum.

11. A program (22a) for a computer of a quality control apparatus (1) configured to inspect quality of an inspection target sample (S) filled in a transparent container (B), the inspection target sample being a lyophilized product or a powdery pharmaceutical product, the quality inspection apparatus including: an illuminator (13) that illuminates a bottom surface of the transparent container; and a multispectral light receiver (12) that obtains an image of a lateral surface of the transparent container, the program causing the computer to:

calculate an absorption spectrum at multiple wavelengths, based on a light reception result by the multispectral light receiver; and

detect an inspection target component contained in the inspection target sample, based on the calculated absorption spectrum.

# FIG.1

| INSPECTION UNIT 10 | PROCESSING DEVICE 20 |

- 11 ROTATABLE MEMBER
- 12 IMAGER
- 13 ILLUMINATION UNIT
  - 131 ILLUMINATION LIGHT
  - 132 LIGHT SOURCE
- 21 CONTROLLER
- 22 STORAGE
  - 22a PROGRAM
- 23 COMMUNICATION PART
- 24 OPERATION PART
- 25 DISPLAY

1

# FIG.2

# FIG.3

# FIG.4

# FIG.5

```
( QUALITY INSPECTION PROCESS )
            │
            ▼
┌─────────────────────────┐  ─ S1
│    DARK MEASUREMENT     │
└─────────────────────────┘
            │
            ▼
┌─────────────────────────┐  ─ S2
│ MEASUREMENT OF REFERENCE │
│         SAMPLE          │
└─────────────────────────┘
            │
            ▼
┌─────────────────────────┐  ─ S3
│ MEASUREMENT OF INSPECTION │
│      TARGET SAMPLE      │
└─────────────────────────┘
            │
            ▼
┌─────────────────────────┐  ─ S4
║  ABSORPTION SPECTRUM    ║
║   CALCULATION PROCESS   ║
└─────────────────────────┘
            │
            ▼
┌─────────────────────────┐  ─ S5
│     PRE-PROCESSING      │
└─────────────────────────┘
            │
            ▼
┌─────────────────────────┐  ─ S6
│    DETECTION PROCESS    │
└─────────────────────────┘
            │
            ▼
     (      END      )
```

# FIG.6

```
( ABSORPTION SPECTRUM
  CALCULATION PROCESS )
            │
            ▼
┌─────────────────────────┐  ─ S11
│  DARK CORRECTION PROCESS │
└─────────────────────────┘
            │
            ▼
┌─────────────────────────┐  ─ S12
│   CALCULATE DIFFUSE     │
│    TRANSMITTANCE        │
└─────────────────────────┘
            │
            ▼
┌─────────────────────────┐  ─ S13
│  CALCULATE ABSORPTION   │
│       SPECTRUM          │
└─────────────────────────┘
            │
            ▼
     (      END      )
```

# FIG.7

INCIDENT LIGHT $I_0$

# FIG.8

# FIG.9

(i)

STANDARD NORMAL VARIATE ABSORBANCE vs WAVELENGTH [nm]

MATCHING RATE IS LESS THAN PREDETERMINED THRESHOLD

(ii)

STANDARD NORMAL VARIATE ABSORBANCE vs WAVELENGTH [nm]

MATCHING RATE IS GREATER THAN OR EQUAL TO PREDETERMINED THRESHOLD

(iii)

STANDARD NORMAL VARIATE ABSORBANCE vs WAVELENGTH [nm]

# FIG.10

# FIG.11A

# FIG.11B

Europäisches
Patentamt

European
Patent Office

Office européen
des brevets

# EUROPEAN SEARCH REPORT

**Application Number**

EP 25 16 2212

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP 3 351910 B2 (EISAI CO LTD) 3 December 2002 (2002-12-03) | 1-5,7-11 | INV. G01N21/31 G01N21/35 G01N21/51 |
| A | * paragraphs [0001], [0018], [0024]; figure 5 * | 6 | |
| Y | US 2023/038654 A1 (MILNE GRAHAM F [US] ET AL) 9 February 2023 (2023-02-09) | 1-5,7-11 | |
| A | * paragraphs [0004], [0074], [0247] - [0250]; figures 8,9,38,45 * | 6 | |
| Y | WO 2007/063840 A1 (EISAI R&D MAN CO LTD [JP]; TAKEUCHI TOMOHIKO [JP]) 7 June 2007 (2007-06-07) * paragraphs [0014] - [0017] * | 4 | |
| A | BROUCKAERT DAVINIA ET AL: "Potential of Near-Infrared Chemical Imaging as Process Analytical Technology Tool for Continuous Freeze-Drying", ANALYTICAL CHEMISTRY, vol. 90, no. 7, 3 April 2018 (2018-04-03), pages 4354-4362, XP055820543, DOI: 10.1021/acs.analchem.7b03647 Retrieved from the Internet: URL:https://pubs.acs.org/doi/pdf/10.1021/acs.analchem.7b03647> * abstract; figure 1- * | 1-11 | **TECHNICAL FIELDS SEARCHED (IPC)**<br><br>G01N |

-/--

The present search report has been drawn up for all claims

| Place of search | Date of completion of the search | Examiner |
|---|---|---|
| Munich | 15 July 2025 | Meacher, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 25 16 2212

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CARNEIRO RENATO L. ET AL: "A quantitative method using near infrared imaging spectroscopy for determination of surface composition of tablet dosage forms: an example of spirolactone tablets", JOURNAL OF THE BRAZILIAN CHEMICAL SOCIETY, vol. 23, no. 8, 1 August 2012 (2012-08-01), pages 1570-1576, XP093296163, ISSN: 0103-5053, DOI: 10.1590/S0103-50532012005000022 * abstract * * page 1571, right-hand column, lines 3-5 * | 5 | |
| A | ROGGO ET AL: "A review of near infrared spectroscopy and chemometrics in pharmaceutical technologies", JOURNAL OF PHARMACEUTICAL AND BIOMEDICAL ANALYSIS, ELSEVIER B.V, AMSTERDAM, NL, vol. 44, no. 3, 10 July 2007 (2007-07-10), pages 683-700, XP022145323, ISSN: 0731-7085, DOI: 10.1016/J.JPBA.2007.03.023 * the whole document * | 1-11 | |
| A | BR?LLS MIKAEL ET AL: "In-Situ Near-Infrared Spectroscopy Monitoring of the Lyophilization Process", PHARMACEUTICAL RESEARCH, vol. 20, no. 3, 31 March 2003 (2003-03-31), pages 494-499, XP093294981, * the whole document * | 1-11 | TECHNICAL FIELDS SEARCHED (IPC) |

-/--

The present search report has been drawn up for all claims

| Place of search | Date of completion of the search | Examiner |
|---|---|---|
| Munich | 15 July 2025 | Meacher, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROGGO Y ET AL: "Infrared hyperspectral imaging for qualitative analysis of pharmaceutical solid forms", ANALYTICA CHIMICA ACTA, ELSEVIER, AMSTERDAM, NL, vol. 535, no. 1-2, 11 April 2005 (2005-04-11), pages 79-87, XP027730736, ISSN: 0003-2670 [retrieved on 2005-04-11] * the whole document * | 1-11 | |

-----

TECHNICAL FIELDS
SEARCHED      (IPC)

The present search report has been drawn up for all claims

| Place of search | Date of completion of the search | Examiner |
|---|---|---|
| Munich | 15 July 2025 | Meacher, David |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 16 2212

This annex lists the patent family members relating to the patent documents cited in the above-mentioned European search report.
The members are as contained in the European Patent Office EDP file on
The European Patent Office is in no way liable for these particulars which are merely given for the purpose of information.

15-07-2025

| Patent document cited in search report | | Publication date | Patent family member(s) | | Publication date |
|---|---|---|---|---|---|
| JP 3351910 | B2 | 03-12-2002 | EP | 0701117 A2 | 13-03-1996 |
| | | | JP | 3351910 B2 | 03-12-2002 |
| | | | JP | H0875671 A | 22-03-1996 |
| | | | KR | 960011406 A | 20-04-1996 |
| | | | TW | 284684 B | 01-09-1996 |
| | | | US | 5719679 A | 17-02-1998 |
| US 2023038654 | A1 | 09-02-2023 | AR | 087733 A1 | 16-04-2014 |
| | | | AU | 2012302036 A1 | 13-02-2014 |
| | | | AU | 2016100220 A4 | 31-03-2016 |
| | | | BR | 112014003321 A2 | 14-03-2017 |
| | | | CA | 2843016 A1 | 07-03-2013 |
| | | | CA | 3048942 A1 | 07-03-2013 |
| | | | CA | 3134342 A1 | 07-03-2013 |
| | | | CL | 2014000374 A1 | 27-06-2014 |
| | | | CN | 103765191 A | 30-04-2014 |
| | | | CN | 105136649 A | 09-12-2015 |
| | | | CN | 106442279 A | 22-02-2017 |
| | | | DK | 2751543 T3 | 22-07-2024 |
| | | | EA | 201490169 A1 | 29-08-2014 |
| | | | EA | 201790956 A2 | 29-09-2017 |
| | | | EP | 2751543 A1 | 09-07-2014 |
| | | | EP | 4365836 A2 | 08-05-2024 |
| | | | ES | 2982079 T3 | 14-10-2024 |
| | | | FI | 2751543 T3 | 11-07-2024 |
| | | | HK | 1199301 A1 | 26-06-2015 |
| | | | IL | 261825 A | 31-10-2018 |
| | | | IL | 286529 A | 31-10-2021 |
| | | | IL | 304646 A | 01-09-2023 |
| | | | JP | 6018208 B2 | 02-11-2016 |
| | | | JP | 6302017 B2 | 28-03-2018 |
| | | | JP | 6368341 B2 | 01-08-2018 |
| | | | JP | 6590876 B2 | 16-10-2019 |
| | | | JP | 6902077 B2 | 14-07-2021 |
| | | | JP | 7216146 B2 | 31-01-2023 |
| | | | JP | 2014525583 A | 29-09-2014 |
| | | | JP | 2016197130 A | 24-11-2016 |
| | | | JP | 2016197131 A | 24-11-2016 |
| | | | JP | 2017201335 A | 09-11-2017 |
| | | | JP | 2019215376 A | 19-12-2019 |
| | | | JP | 2021167821 A | 21-10-2021 |
| | | | KR | 20140060300 A | 19-05-2014 |
| | | | KR | 20190057426 A | 28-05-2019 |
| | | | KR | 20190132579 A | 27-11-2019 |
| | | | KR | 20200121917 A | 26-10-2020 |
| | | | KR | 20210107146 A | 31-08-2021 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 25 16 2212

This annex lists the patent family members relating to the patent documents cited in the above-mentioned European search report.
The members are as contained in the European Patent Office EDP file on
The European Patent Office is in no way liable for these particulars which are merely given for the purpose of information.

15-07-2025

| Patent document cited in search report | Publication date | Patent family member(s) | Publication date |
|---|---|---|---|
| | | KR 20220116080 A | 19-08-2022 |
| | | PL 2751543 T3 | 19-08-2024 |
| | | SG 10201606760T A | 28-10-2016 |
| | | SG 10201806093U A | 30-08-2018 |
| | | TW 201315992 A | 16-04-2013 |
| | | TW 201721124 A | 16-06-2017 |
| | | TW 201721130 A | 16-06-2017 |
| | | TW 201903382 A | 16-01-2019 |
| | | TW 202119011 A | 16-05-2021 |
| | | TW 202242379 A | 01-11-2022 |
| | | US 2014177932 A1 | 26-06-2014 |
| | | US 2016379376 A1 | 29-12-2016 |
| | | US 2016379377 A1 | 29-12-2016 |
| | | US 2016379378 A1 | 29-12-2016 |
| | | US 2018150965 A1 | 31-05-2018 |
| | | US 2021019905 A1 | 21-01-2021 |
| | | US 2023038654 A1 | 09-02-2023 |
| | | WO 2013033253 A1 | 07-03-2013 |
| | | ZA 201400737 B | 27-01-2016 |
| WO 2007063840 A1 | 07-06-2007 | EP 1956362 A1 | 13-08-2008 |
| | | JP WO2007063840 A1 | 07-05-2009 |
| | | US 2010000111 A1 | 07-01-2010 |
| | | WO 2007063840 A1 | 07-06-2007 |

EPO FORM P0459

page 2 of 2

**EP 4 617 642 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3351910 B

- JP 2007063840 A